# EUROPÄISCHE PATENTANMELDUNG

(11) **EP 4 679 120 A1**
(43) Veröffentlichungstag der Anmeldung: **14.01.2026**
(21) Anmeldenummer: 24188010.3
(22) Anmeldetag: 11.07.2024
(51) Int. Cl.: G01R 33/28

(54) **PATIENTENRUFBALL, MAGNETRESONANZVORRICHTUNG MIT EINEM PATIENTENRUFBALL UND VERFAHREN ZU EINEM VERWENDEN EINES PATIENTENRUFBALLS FÜR EINE MAGNETRESONANZUNTERSUCHUNG**

(71) Anmelder: Siemens Healthineers AG, 91301 Forchheim (DE)
(72) Erfinder: Biber, Stephan, 91056 Erlangen (DE)
(74) Vertreter: Siemens Healthineers Patent Attorneys

(57) **Zusammenfassung**

Die Erfindung geht aus von einem Patientenrufball, der zu einer Kommunikation eines Patienten während einer medizinischen Magnetresonanzuntersuchung mit einem medizinischen Bedienpersonal ausgebildet ist, umfassend einen Betätigungsbereich, der zu einem Betätigen, insbesondere zu einem Drücken, durch einen Patienten ausgebildet ist, und eine Signaleinheit, die dazu ausgebildet ist, bei einem Drücken des Betätigungsbereichs ein Kommunikationssignal auszusenden, wobei der Patientenrufball eine Magnetfeldsensoreinheit mit einem Magnetfeldsensor und einer Aktivierungseinheit aufweist, wobei die Aktivierungseinheit zu einer Aktivierung der Signaleinheit ausgebildet ist, sobald ein von dem Magnetfeldsensor erfasster Wert eines Magnetfelds größer ist als ein Grenzwert.

## Beschreibung

Die vorliegende Erfindung betrifft ein einen Patientenrufball, der zu einer Kommunikation eines Patienten während einer medizinischen Bildgebungsvorrichtung mit einem medizinischen Bedienpersonal ausgebildet ist, umfassend einen Betätigungsbereich, der zu einem Betätigen, insbesondere zu einem Drücken, durch einen Patienten ausgebildet ist, und eine Signaleinheit, die dazu ausgebildet ist, bei einem Drücken des Betätigungsbereich ein Signal auszusenden. Des Weiteren betrifft die vorliegende Erfindung eine Magnetresonanzvorrichtung mit einem Patientenrufball. Ferner geht die Erfindung aus von einem Verfahren zu einem Verwenden eines Patientenrufballs für eine Magnetresonanzuntersuchung.

Unabhängig vom grammatikalischen Geschlecht eines bestimmten Begriffes sind Personen mit männlicher, weiblicher oder anderer Geschlechteridentität mit umfasst.

Während einer Magnetresonanzuntersuchung befindet sich der Patient in einem Patientenaufnahmebereich einer Scannereinheit einer Magnetresonanzvorrichtung. Die Scannereinheit ist innerhalb eines Untersuchungsraums angeordnet, wobei der Untersuchungsraum hinsichtlich einer HF-Strahlung nach außen abgeschirmt ausgebildet ist. Ein die Magnetresonanzuntersuchung betreuendes medizinisches Bedienpersonal, beispielsweise ein Arzt, dagegen befindet sich während der Magnetresonanzuntersuchung in einem Kontrollraum, der getrennt von dem Untersuchungsraum ausgebildet ist. Für eine Mitteilung von Informationen des Patienten an das medizinische Bedienpersonal während der Magnetresonanzuntersuchung ist bisher ein Patientenrufball bekannt. Diesen Patientenrufball hält der Patient während der Magnetresonanzuntersuchung in der Hand und kann diesen bei Bedarf drücken, wobei durch Drücken des Patientenrufballs ein Signal an das medizinische Bedienpersonal gesendet wird. Durch das Drücken kann der Patient auf eine Notsituation aufmerksam machen und/oder um Aufmerksamkeit des medizinischen Bedienpersonals bitten.

Bisherige Patientenrufbälle sind vorwiegend pneumatisch ausgeführt. Dabei weisen die Patientenrufbälle einen Luftschlauch auf, wobei mittels des Luftschlauchs durch Drücken des Patientenrufballs ein pneumatisches Signal zu einem Sensor am Patiententisch übermittelt wird. Der Senor wandelt das pneumatische Signal in eine elektrisches Signal, welches dann zu Ausgabe an das medizinische Bedienpersonal gebracht wird.

Ein Luftschlauch eines Patientenrufballs muss sehr sorgfältig für eine Magnetresonanzuntersuchung auf dem Patiententisch positioniert werden, um während eine Einfahrvorgangs und/oder Ausfahrvorgangs des Patiententischs ein Herabrutschen des Luftschlauchs zu verhindern. Ein herabhängender Luftschlauch kann bei einer Fahrbewegung des Patiententischs zwischen dem Patiententisch und einer Umhausung des Patientenaufnahmebereichs eingeklemmt und dabei beschädigt werden.

Für ein sicheres Bewegen des Patiententischs, bei dem ein Einklemmen des Luftschlauchs verhindert ist, können drahtlose und/oder kabellose Patientenrufbälle verwendet werden. Sofern diese ein Hochfrequenz-basiertes Verfahren zur Übertragung von Signalen und/oder Informationen umfassen, weisen diese das Problem auf, dass sie als Funkgerät zugelassen werden müssen oder EMV(Elektromagnetische Verträglichkeit)-Grenzwerte einhalten müssen. Dadurch dass der Patientenrufball nicht mehr mechanisch mit der Scannereinheit und/oder dem Patiententisch verbunden ist, kann der Patientenrufball überall mitgenommen werden, auch außerhalb des Untersuchungsraums und muss daher auch außerhalb des HF-geschirmtes Untersuchungsraums erforderliche Grenzwerte einhalten.

Der vorliegenden Erfindung liegt insbesondere die Aufgabe zugrunde, eine Beeinträchtigung von Bauteilen und/oder Geräten, die vorzugsweise außerhalb des Untersuchungsraums angeordnet sind, durch den Patientenrufball zu minimieren. Die Aufgabe wird durch die Merkmale der unabhängigen Ansprüche gelöst. Vorteilhafte Ausgestaltungen sind in den Unteransprüchen beschrieben.

Ein erster Aspekt der Erfindung geht aus von einem Patientenrufball, der zu einer Kommunikation einer Patienten während einer medizinischen Magnetresonanzuntersuchung mit einem medizinischen Bedienpersonal ausgebildet ist, umfassend einen Betätigungsbereich, der zu einem Betätigen, insbesondere einem Drücken, durch einen Patienten ausgebildet ist, und eine Signaleinheit, die dazu ausgebildet ist, bei einem Drücken des Betätigungsbereichs ein Kommunikationssignal auszusenden. Es wird vorgeschlagen, dass der Patientenrufball eine Magnetfeldsensoreinheit mit einem Magnetfeldsensor und einer Aktivierungseinheit umfasst, wobei die Aktivierungseinheit zu einer Aktivierung der Signaleinheit ausgebildet ist, sofern ein von dem Magnetfeldsensor erfasster Wert eines Magnetfelds größer ist als ein Aktivierungsgrenzwert.

Bevorzugt ist der Patientenrufball als kabelloser und/oder drahtloser Patientenrufball ausgebildet. Dabei kann eine kabellose und/oder drahtlose Datenübertragung über eine Funkverbindung, beispielsweise eine Bluetooth-Verbindung, und/oder eine durch Aussenden von Hochfrequenz-Signalen und/oder durch Aussenden von Pilotton-Signalen und/oder weiterer, dem Fachmann als sinnvoll erscheinender kabellosen Datenübertragungsmethoden erfolgen. Vorzugsweise weist die Signaleinheit zu einem Aussenden eines Kommunikationssignals eine Sendeantenne auf. Dabei sendet die Signaleinheit das Kommunikationssignal bevorzugt auf Basis von elektromagnetischen Wellen oder elektromagnetischer Kopplung.

Der Patientenrufball weist einen Betätigungsbereich auf, wobei der Betätigungsbereich zumindest eine Taste und/oder zumindest einen Drückbereich aufweist, wobei die zumindest eine Taste und/oder der zumindest eine Drückbereich zu einem Drücken durch einen Benutzer, bevorzugt den Patienten, ausgebildet ist. Bevorzugt weist der Patientenrufball eine einzige Taste und/oder einen einzigen Drückbereich auf, um ein Kommunikationssignal auszusenden. Der Patientenrufball weist bevorzugt eine Größe auf, die ein Halten, insbesondere ein Umschließen, des Patientenrufballs mit einer Hand, insbesondere einer einzigen Hand, des Patienten ermöglicht. Bevorzugt ist mit einer Hand des Patienten ein Umgreifen des Patientenrufballs, insbesondere ein Umgreifen von mindestens 50% des Patientenrufballs und bevorzugt von mindestens 70% des Patientenrufballs, möglich.

Der Patientenrufball kommuniziert bevorzugt mit einer Kommunikationseinheit, die innerhalb des Kontrollraums angeordnet ist und zu einer Ausgabe des Kommunikationssignals an einen Benutzer, insbesondere das die Magnetresonanzuntersuchung betreuendes medizinisches Bedienpersonal, ausgebildet ist. Das Kommunikationssignal kann dem Benutzer, insbesondere dem medizinischen Bedienpersonal, ein Unwohlsein und/oder eine Notsituation des Patienten während der Magnetresonanzuntersuchung mitteilen. Bei Betätigen, insbesondere Drücken, des Betätigungsbereichs des Patientenrufballs erhält das medizinische Bedienpersonal eine entsprechende Ausgabeinformation an einem Kommunikationselement, insbesondere einem Ausgabeelement, der Kommunikationseinheit.

Die Magnetfeldsensoreinheit umfasst bevorzugt den Magnetfeldsensor und die Aktivierungseinheit. Der Magnetfeldsensor kann beispielsweise eine Hallsensor umfassen. Zudem ist der Magnetfeldsensor zu einer Erfassung eines Magnetfelds ausgebildet. Derart kann ermittelt werden, ob sich der Patientenrufball in der Nähe zur Magnetresonanzvorrichtung, insbesondere einer Scannereinheit der Magnetresonanzvorrichtung befindet. Ferner kann die Magnetfeldsensoreinheit einen 3D-Magnetfeldsensor umfassen, beispielsweise einen 3D-Hallsensor, so dass zudem eine Erfassung einer Position des Patientenrufballs möglich ist.

Die Aktivierungseinheit ist zunächst zu einem Vergleich des von dem Magnetfeldsensor erfassten Magnetfelds mit einem Grenzwert, insbesondere einem vorgegebenen Aktivierungsgrenzwert, ausgebildet. Der Aktivierungsgrenzwert ist vorzugsweise an die Magnetresonanzvorrichtung, insbesondere an ein Magnetfeld der Magnetresonanzvorrichtung angepasst. Dabei können für unterschiedliche Magnetresonanzvorrichtungen mit unterschiedlichen Magnetfeldstärken auch unterschiedliche Aktivierungsgrenzwerte für den Patientenrufball vorhanden sein. Sobald von dem Magnetfeldsensor ein Magnetfeld erfasst wird, dessen Wert größer als der von der Aktivierungseinheit vorgegebene Aktivierungsgrenzwert ist, erfolgt von der Aktivierungseinheit eine Aktivierung der Signaleinheit. Vorzugsweise ist der Aktivierungsgrenzwert derart ausgebildet, dass nur innerhalb des Untersuchungsraums ein Überschreiten des Aktivierungsgrenzwert durch einen gemessenen Wert der Magnetfeldstärke möglich ist. Bevorzugt ist dabei nur innerhalb eines Patientenaufnahmebereichs einer Magnetresonanzvorrichtung oder in geringem Abstand zur Scannereinheit der Magnetresonanzvorrichtung, beispielsweise ein Abstand von kleiner 2 m, bevorzugt kleiner 1,5m zur Scannereinheit, ein Überschreiten des Aktivierungsgrenzwert durch einen gemessenen Wert der Magnetfeldstärke und damit eine Aktivierung der Signaleinheit möglich.

Die Aktivierungseinheit weist hierzu eine Steuereinheit und/oder eine Recheneinheit auf. Die Steuereinheit und/oder Recheneinheit kann dabei eine entsprechende Schaltung und/oder auch ein entsprechendes Rechenmodul aufweisen. Bevorzugt erfolgt die Aktivierung der Signaleinheit automatisch von der Aktivierungseinheit.

Die Erfindung weist den Vorteil auf, dass die Signaleinheit nur in innerhalb des Magnetfelds der Magnetresonanzvorrichtung und damit in einer Nähe zur Scannereinheit der Magnetresonanzvorrichtung aktiviert werden kann. Damit ist auch sichergestellt, dass der Patientenrufball nur innerhalb des Untersuchungsraums aktiviert werden kann. Ein versehentliches Betätigen, insbesondere Drücken, des Betätigungsbereichs, wenn der Patientenrufball außerhalb des Untersuchungsraums angeordnet ist, führt damit zu keinem Aussenden eines Kommunikationssignals, da die Signaleinheit in einem derartigen Fall nicht aktiviert ist und auch nicht aktiviert werden kann. Hierdurch können Störungen auf andere Geräte, die durch Aussenden eine Kommunikationssignals außerhalb des Untersuchungsraums hervorgerufen werden würden, vorteilhaft verhindert werden. Ein weiterer Vorteil ist, dass der Untersuchungsraum bezüglich Hochfrequenzstrahlung nach außen abgeschirmt ist, so dass eine Aktivierung der Signaleinheit und ein Aussenden von Kommunikationssignalen nur innerhalb eines geschirmten Umfelds möglich ist. Durch eine Schirmkabine, die um den Untersuchungsraum angeordnet ist, kann eine Dämpfung eines von dem Patientenrufball ausgesendeten Kommunikationssignals um mindestens 10 dB bis zu 40 dB erfolgen. Derart kann ein hoher Sicherheitsstandard für eine Magnetresonanzvorrichtung mit einem drahtlosen und/oder kabellosen Patientenrufball bereitgestellt werden.

In einer vorteilhaften Weiterbildung des erfindungsgemäßen Patientenrufballs wird vorgeschlagen, dass die Signaleinheit zu einem Aussenden eines Registrierungssignals ausgebildet ist. Bevorzugt ist die Signaleinheit zu Beginn einer Aktivierung durch die Aktivierungseinheit zu einem Aussenden des Registrierungssignals ausgebildet. Vorzugsweise unterscheidet sich das Registrierungssignal von dem Kommunikationssignal, so dass die Signaleinheit zu einem Aussenden von zwei unterschiedlichen Signalen, nämlich den Kommunikationssignal und einen davon unterschiedlichen Registrierungssignal, ausgebildet ist. Das Registrierungssignal kann dabei von einer Kommunikationseinheit, die dem medizinischen Bedienpersonal für eine Kommunikation mit dem Patienten während der Magnetresonanzvorrichtung zur Verfügung steht, erfasst werden. Vorzugsweise wird von der Kommunikationseinheit eine Registrierungsinformation basierend auf dem Registrierungssignal an den Benutzer, insbesondere ein medizinisches Bedienpersonal, ausgegeben. Derart kann der Benutzer, insbesondere ein medizinisches Bedienpersonal, über eine erfolgreiche Registrierung und Aktivierung des Patientenrufballs für eine Magnetresonanzuntersuchung informiert werden. Insbesondere zeigt das Registrierungssignal und damit die ausgegebene Registrierungsinformation an den Benutzer, insbesondere dem medizinischen Bedienpersonal, an, dass der Patientenrufball einsatzbereit ist.

In einer vorteilhaften Weiterbildung des erfindungsgemäßen Patientenrufballs wird vorgeschlagen, dass der Patientenrufball eine Ausgabeeinheit umfasst, die zu einer Ausgabe einer Registrierungsinformation ausgebildet ist. Bevorzugt kann die Registrierungsinformation von der Aktivierungseinheit generiert und zur Ausgabe der Ausgabeeinheit bereitgestellt werden. Dabei kann die Ausgabeeinheit eine optische Ausgabeeinheit zur optischen Ausgabe der Registrierungsinformation oder eine akustische Ausgabeeinheit zur akustischen Ausgabe der Registrierungsinformation umfassen. Beispielsweise kann die optische Ausgabeeinheit durch eine einfache Farbänderung einer Lichtanzeige eine erfolgreiche Registrierung und/oder Aktivierung des Patientenrufballs dem Benutzer und/oder dem Patienten angezeigt werden. Umfasst die Ausgabeeinheit eine akustischen Ausgabeeinheit, kann beispielsweise durch Aussenden eines Signaltons eine erfolgreiche Registrierung und/oder Aktivierung des Patientenrufballs dem Benutzer und/oder dem Patienten angezeigt werden.

In einer vorteilhaften Weiterbildung des erfindungsgemäßen Patientenrufballs wird vorgeschlagen, dass die Signaleinheit eine Empfangseinheit zu einem Empfangen eine Bestätigungssignals aufweist. Vorzugsweise wird das Bestätigungssignal von der Kommunikationseinheit, die dem medizinischen Bedienpersonal während einer Magnetresonanzuntersuchung für eine Kommunikation mit dem Patienten zur Verfügung steht, an den Patientenrufball, insbesondere an die Signaleinheit des Patientenrufballs, nach Erhalt des Registrierungssignals gesendet. Das empfangene Bestätigungssignal kann zudem direkt über die Ausgabeeinheit des Patientenrufballs ausgegeben werden, so dass ein Benutzer, beispielsweise ein medizinisches Bedienpersonal, bereits während der Vorbereitung des Patienten für eine Magnetresonanzuntersuchung eine Information erhält, dass der Patientenrufball erfolgreich aktiviert und registriert wurde. Dies kann auch einen Arbeitsablauf für das medizinische Bedienpersonal vereinfachen, da zur Kontrolle einer erfolgreichen Aktivierung und Registrierung des Patientenrufballs der Untersuchungsraum von dem medizinischen Bedienpersonal nicht verlassen werden muss.

In einer vorteilhaften Weiterbildung des erfindungsgemäßen Patientenrufballs wird vorgeschlagen, dass die Aktivierungseinheit zu einer Deaktivierung der Signaleinheit ausgebildet ist, sobald ein von dem Magnetfeldsensor erfasster Wert eines Magnetfelds kleiner ist als ein Deaktivierungsgrenzwert. Der Aktivierungsgrenzwert, auf den eine Aktivierung der Signaleinheit durch die Aktivierungseinheit basiert, kann dabei der gleiche sein, wie der Deaktivierungsgrenzwert, auf den eine Deaktivierung der Signaleinheit durch die Aktivierungseinheit basiert. Zudem können die beiden Grenzwerte auch unterschiedlich ausgebildet sein. Ferner kann der Aktivierungsgrenzwert für eine Aktivierung der Signaleinheit größer sein als der Deaktivierungsgrenzwert für eine Deaktivierung der Signaleinheit. Die Deaktivierung der Signaleinheit erfolgt bevorzugt automatisch durch die Aktivierungseinheit. Nach eine Deaktivierung befindet sich die Signaleinheit in einem deaktiven Modus oder Sicherheitsmodus, so dass bei einem Betätigen, insbesondere einem Drücken, des Betätigungsbereichs ein Aussenden eines Signal nicht möglich ist oder verhindert wird. Hierdurch können Beeinträchtigungen weiterer Geräte durch den Patientenrufball, beispielsweise durch ein Drücken des Betätigungsbereichs in einem deaktiven Modus oder dem Sicherheitsmodus der Signaleinheit, vorteilhaft verhindert werden. Insbesondere kann hierbei ein Aussenden eines Signals durch die Signaleinheit außerhalb eines Bereichs mit einem hohen Magnetfeld und/oder außerhalb des Untersuchungsbereichs vorteilhaft verhindert werden.

Ein weiterer Aspekt der Erfindung geht aus von einer Magnetresonanzvorrichtung mit einer ersten Kommunikationseinheit, die innerhalb eine Kontrollraums angeordnet ist, und einer zweiten Kommunikationseinheit, die innerhalb eines Untersuchungsraums angeordnet ist, wobei die zweite Kommunikationseinheit einen Patientenrufball aufweist und wobei die erste Kommunikationseinheit und der Patientenrufball zu einem Austausch von Kommunikationssignalen ausgebildet sind.

Die Magnetresonanzvorrichtung umfasst bevorzugt eine medizinische und/oder diagnostische Magnetresonanzvorrichtung, die zu einem Erfassen von medizinischen und/oder diagnostischen Bilddaten, insbesondere medizinischen und/oder diagnostischen Magnetresonanzbilddaten, eines Patienten ausgelegt und/oder ausgebildet ist. Die Magnetresonanzvorrichtung umfasst hierzu eine Scannereinheit, die innerhalb des Untersuchungsraums angeordnet ist. Die Scannereinheit umfasst bevorzugt eine Magneteinheit zur Erfassung der medizinischen und/oder diagnostischen Bilddaten. Vorteilhafterweise umfasst hierbei die Scannereinheit, insbesondere die Magneteinheit, einen Grundmagneten, ein Gradientenspuleneinheit und eine Hochfrequenzantenneneinheit. Die Hochfrequenzantenneneinheit ist fest innerhalb der Scannereinheit angeordnet und zur Aussendung eines Anregungspulses ausgelegt und/oder ausgebildet.

Der Grundmagnet ist zur Erzeugung eines homogenen Grundmagnetfelds mit einer definierten Magnetfeldstärke, wie beispielsweise mit einer Magnetfeldstärke von 3 T oder 1,5 T usw., ausgebildet. Insbesondere ist der Grundmagnet zur Erzeugung eines starken und konstanten Grundmagnetfelds ausgebildet. Das homogene Grundmagnetfeld ist bevorzugt innerhalb eines Patientenaufnahmebereichs der Magnetresonanzvorrichtung angeordnet und/oder vorzufinden. Die Gradientenspuleneinheit ist zu einer Erzeugung von Magnetfeldgradienten, die für eine Ortskodierung während einer Bildgebung verwendet werden, ausgebildet.

Der Patientenaufnahmebereich der Magnetresonanzvorrichtung ist zu einer Aufnahme des Patienten, insbesondere des zu unter-suchenden Bereichs des Patienten, für eine medizinische Magnetresonanzuntersuchung ausgelegt und/oder ausgebildet. Der Patientenaufnahmebereich umfasst bevorzugt den Bereich, der dem Patienten während einer Magnetresonanzuntersuchung zur Verfügung steht. Beispielsweise ist hierzu der Patientenaufnahmebereich zylinderförmig ausgebildet und/oder zylinderförmig von der Scannereinheit, insbesondere der Magneteinheit, der Magnetresonanzvorrichtung umgeben.

Innerhalb des Patientenaufnahmebereichs ist bevorzugt ein Field of View (FoV) und ein Isozentrum der Magnetresonanzvorrichtung angeordnet. Das FoV umfasst bevorzugt einen Erfassungsbereich der Magnetresonanzvorrichtung, innerhalb dessen die Bedingungen für eine Erfassung von medizinischen Bilddaten, insbesondere von Magnetresonanzbilddaten, innerhalb des Patientenaufnahmebereichs vorliegen, wie beispielsweise ein homogenes Grundmagnetfeld. Das Isozentrum der Magnetresonanzvorrichtung umfasst bevorzugt den Bereich und/oder Punkt innerhalb der Magnetresonanzvorrichtung, der die optimalen und/oder idealen Bedingungen für die Erfassung von medizinischen Bilddaten aufweist. Insbesondere umfasst das Isozentrum den homogensten Magnetfeldbereich innerhalb der Magnetresonanzvorrichtung.

Für eine Positionierung des Patienten, insbesondere des zu untersuchenden Bereichs des Patienten, innerhalb des Patientenaufnahmebereich weist die Magnetresonanzvorrichtung die Patientenlagerungsvorrichtung auf. Die Patientenlagerungsvorrichtung ist zu einer Lagerung des Patienten innerhalb des Patientenaufnahmebereichs ausgebildet. Die Patientenlagerungsvorrichtung weist hierzu einen bewegbaren Patiententisch auf, der insbesondere innerhalb des Patientenaufnahmebereichs der Magnetresonanzvorrichtung bewegbar ausgebildet ist. Bevorzugt ist hierbei der Patiententisch in Längsrichtung des Patientenaufnahmebereichs und/oder in z-Richtung innerhalb des Patientenaufnahmebereichs bewegbar ausgebildet. Für eine Magnetresonanzuntersuchung wird der Patient auf dem Patiententisch der Patientenlagerungsvorrichtung positioniert und für die Magnetresonanzuntersuchung erforderliche Zusatzeinheiten, insbesondere der Patientenrufball, ebenfalls auf dem Patiententisch oder am Patienten positioniert und in den Patientenaufnahmebereich eingefahren.

In dem Kontrollraum befindet sich während der Magnetresonanzuntersuchung das medizinische Bedienpersonal. Der Kontrollraum ist zu einer Kontrolle und/oder Überwachung von Magnetresonanzuntersuchungen mit einer Benutzerschnittstelle und der ersten Kommunikationseinheit ausgestattet. Zudem kann auch innerhalb des Kontrollraums ein Host-Rechner der Magnetresonanzvorrichtung angeordnet sein. Der Kontrollraum ist getrennt zum Untersuchungsraum, in dem die Scannereinheit der Magnetresonanzvorrichtung angeordnet ist, ausgebildet, insbesondere ist der Untersuchungsraum hinsichtlich einer HF-Strahlung von dem Kontrollraum abgeschirmt.

Die erste Kommunikationseinheit umfasst eine Ausgabeeinheit, die zu einer Ausgabe eines Kommunikationssignals ausgebildet ist. Die Ausgabeeinheit ist bevorzugt als optische Ausgabeeinheit mit einer Anzeige und/oder einem Display usw. ausgebildet. Alternativ hierzu kann die Ausgabeeinheit auch eine akustische Ausgabeeinheit umfassen. Neben einer Ausgabeeinheit kann die erste Kommunikationseinheit auch eine Eingabeeinheit umfassen, die zu einer Eingabe einer Information an den Patienten ausgebildet ist. Die zweite Kommunikationseinheit umfasst den Patientenrufball. Neben dem Patientenrufball kann die zweite Kommunikationseinheit zudem weitere Kommunikationselemente umfassen, beispielsweise optische und/oder akustische Ausgabeelemente, wie insbesondere Kopfhörer und/oder ein Display.

Eine derartige Magnetresonanzvorrichtung weist den Vorteil auf, dass die Signaleinheit nur in innerhalb des Magnetfelds der Magnetresonanzvorrichtung und damit in einer Nähe zur Scannereinheit der Magnetresonanzvorrichtung aktiviert werden kann. Damit ist auch sichergestellt, dass der Patientenrufball nur innerhalb des Untersuchungsraums aktiviert werden kann. Ein versehentliches Betätigen, insbesondere Drücken, des Betätigungsbereichs, wenn der Patientenrufball außerhalb des Untersuchungsraums angeordnet ist, führt damit zu keinem Aussenden eines Kommunikationssignals, da die Signaleinheit in einem derartigen Fall nicht aktiviert ist und auch nicht aktiviert werden kann. Hierdurch können Störungen auf andere Geräte, die durch Aussenden eine Kommunikationssignals außerhalb des Untersuchungsraums, hervorgerufen werden würden, vorteilhaft verhindert werden. Ein weiterer Vorteil ist, dass der Untersuchungsraum bezüglich Hochfrequenzstrahlung nach außen abgeschirmt ist, so dass eine Aktivierung der Signaleinheit und ein Aussenden von Kommunikationssignalen nur innerhalb eines geschirmten Umfelds möglich ist. Durch eine Schirmkabine, die um den Untersuchungsraum angeordnet ist, kann eine Dämpfung eines von dem Patientenrufball ausgesendeten Kommunikationssignals um mindestens 10 dB bis zu 40 dB erfolgen. Derart kann ein hoher Sicherheitsstandard für eine Magnetresonanzvorrichtung mit einem drahtlosen und/oder kabellosen Patientenrufball bereitgestellt werden.

Die Vorteile der erfindungsgemäßen Magnetresonanzvorrichtung entsprechen im Wesentlichen den Vorteilen des erfindungsgemäßen Patientenrufballs, welche vorab im Detail ausgeführt sind. Hierbei erwähnte Merkmale, Vorteile oder alternative Ausführungsformen können ebenso auf die anderen beanspruchten Gegenstände übertragen werden und umgekehrt.

In einer vorteilhaften Weiterbildung des erfindungsgemäßen Magnetresonanzvorrichtung wird vorgeschlagen, dass die erste Kommunikationseinheit eine Empfangseinheit, die zu einem Empfangen von Signalen des Patientenrufballs ausgebildet ist, und eine Sendeeinheit, die zu einem Senden von Signalen an den Patientenrufball ausgebildet ist, umfasst. Dies ermöglicht einen einfachen Austausch von Signalen zwischen der ersten Kommunikationseinheit und dem Patientenrufball. Insbesondere kann derart zusätzlich zu einem Empfangen von Kommunikationssignalen und/oder Registrierungssignalen durch die erste Kommunikationseinheit auch ein Bestätigungssignal für eine Bestätigung einer Aktivierung und/oder Registrierung an den Patientenrufball gesendet werden.

Ein weiterer Aspekt der Erfindung geht aus von einem Verfahren zu einem Verwenden eines Patientenrufballs für eine Magnetresonanzuntersuchung mit einem Patientenrufball und einer Magnetresonanzvorrichtung, wobei das Verfahren die folgenden Verfahrensschritte umfasst:
- Erfassen eines Werts eines Magnetfelds am Patientenrufball mittels eines Magnetfeldsensors des Patientenrufballs, und
- Aktivieren einer Signaleinheit des Patientenrufballs mittels einer Aktivierungseinheit des Patientenrufballs, sobald der erfasste Wert des Magnetfelds einen Aktivierungsgrenzwert überschreitet.

Für eine Aktivierung der Signaleinheit durch die Aktivierungseinheit muss der Patientenrufball in das Magnetfeld der Magnetresonanzvorrichtung eingebracht werden. Ein Einbringen des

Patientenrufballs in das Magnetfeld der Magnetresonanzvorrichtung erfolgt bevorzugt während einer Vorbereitung eines Patienten für eine Magnetresonanzuntersuchung. Hierbei wird dem Patienten für Notfälle während der Magnetresonanzuntersuchung der Patientenrufball übergeben. Anschließen wird der Patient zusammen mit dem Patientenrufball mittels des Patiententischs in den Patientenaufnahmebereich eingefahren.

Hierdurch kann die Signaleinheit nur in innerhalb des Magnetfelds der Magnetresonanzvorrichtung und damit in einer Nähe zur Scannereinheit der Magnetresonanzvorrichtung aktiviert werden. Damit ist auch sichergestellt, dass der Patientenrufball nur innerhalb des Untersuchungsraums aktiviert werden kann. Ein versehentliches Betätigen, insbesondere Drücken, des Betätigungsbereichs, wenn der Patientenrufball außerhalb des Untersuchungsraums angeordnet ist, führt damit zu keinem Aussenden eines Kommunikationssignals, da die Signaleinheit in einem derartigen Fall nicht aktiviert ist und auch nicht aktiviert werden kann. Hierdurch können Störungen auf andere Geräte, die durch Aussenden eine Kommunikationssignals außerhalb des Untersuchungsraums hervorgerufen werden würden, vorteilhaft verhindert werden. Ein weiterer Vorteil ist, dass der Untersuchungsraum bezüglich Hochfrequenzstrahlung nach außen abgeschirmt ist, so dass eine Aktivierung der Signaleinheit und ein Aussenden von Kommunikationssignalen nur innerhalb eines geschirmten Umfelds möglich ist. Durch eine Schirmkabine, die um den Untersuchungsraum angeordnet ist, kann eine Dämpfung eines von dem Patientenrufball ausgesendeten Kommunikationssignals um mindestens 10 dB bis zu 40 dB erfolgen. Derart kann ein hoher Sicherheitsstandard für eine Magnetresonanzvorrichtung mit einem drahtlosen und/oder kabellosen Patientenrufball bereitgestellt werden.

Die Vorteile des erfindungsgemäßen Verfahrens zu einem Verwenden eines Patientenrufballs für eine Magnetresonanzuntersuchung entsprechen im Wesentlichen den Vorteilen des erfindungsgemäßen Patientenrufballs und der erfindungsgemäßen Magnetresonanzvorrichtung, welche vorab im Detail ausgeführt sind. Hierbei erwähnte Merkmale, Vorteile oder alternative Ausführungsformen können auch auf die anderen beanspruchten Gegenstände übertragen werden und umgekehrt.

In einer vorteilhaften Weiterbildung des erfindungsgemäßen Verfahrens wird vorgeschlagen, dass die Aktivierung der Signaleinheit ein Aussenden eines Registrierungssignals durch die Signaleinheit umfasst, wobei das Registrierungssignal von einer Kommunikationseinheit der Magnetresonanzvorrichtung erfasst wird. Das Registrierungssignal wird dabei zu Beginn der Aktivierung von der Signaleinheit ausgesendet. Das Registrierungssignal ist dabei unterschiedlich ausgebildet zu dem Kommunikationssignal, so dass die Signaleinheit zu einem Aussenden von zwei unterschiedlichen Signalen, nämlich den Kommunikationssignal und einen davon unterschiedlichen Registrierungssignal, ausgebildet ist. Durch das Registrierungssignal kann eine Information bezüglich einer Aktivierung des Patientenrufballs an der Magnetresonanzvorrichtung vorliegen. Damit kann auch ein Benutzer, insbesondere ein medizinisches Bedienpersonal, überprüfen, ob der Patientenrufball einsatzbereit für die anstehende Magnetresonanzuntersuchung ist.

In einer vorteilhaften Weiterbildung des erfindungsgemäßen Verfahrens wird vorgeschlagen, dass zusätzlich zu dem Registrierungssignal eine Rufballinformation an die Kommunikationseinheit der Magnetresonanzvorrichtung übermittelt wird. Vorzugsweise wird die Rufballinformation von der Signaleinheit des Patientenrufballs an die Kommunikationseinheit der Magnetresonanzvorrichtung übermittelt. Die Rufballinformation umfasst bevorzugt eine Typennummer und/oder Seriennummer des Patientenrufballs. Diese Ausgestaltung ermöglicht eine Überprüfung des Patientenrufballs, ob dieser für die Magnetresonanzvorrichtung zugelassen ist oder nicht. Gerade bei Institutionen, beispielsweise Krankenhäusern, mit mehreren Magnetresonanzvorrichtungen stehen auch zumeist mehrere Patientenrufbälle zur Verfügung, so dass eine Überprüfung einer Kompatibilität zwischen Magnetresonanzvorrichtung und Patientenrufball von Vorteil sein kann. Zudem können anhand der bereitgestellten Rufballinformation auch Updates, beispielsweise Software-Updates, für den Patientenrufball bereitgestellt werden.

In einer vorteilhaften Weiterbildung des erfindungsgemäßen Verfahrens wird vorgeschlagen, dass die Registrierungsinformation und/oder die Rufballinformation an der Kommunikationseinheit der Magnetresonanzvorrichtung ausgegeben wird. Vorzugsweise weist hierzu die Kommunikationseinheit eine Ausgabeeinheit, beispielsweise eine optische Ausgabeeinheit, wie ein Display und/oder ein Anzeigeelement, auf. Beispielweise kann die Registrierungsinformation durch ein Einblenden und/oder Anzeigen eines Rufballsymbols und/oder durch ein Ändern einer angezeigten Farbe eines angezeigten und/oder eingeblendeten Rufballsymbols und/oder Ändern einer Darstellung eines angezeigten und/oder eingeblendeten Rufballsymbols dargestellt werden. Alternativ hierzu kann die Ausgabeeinheit auch eine akustische Ausgabeeinheit, beispielsweise einen Lautsprecher, umfassen. Auch kann durch Ausgabe der Rufballinformation, insbesondere eine optische Ausgabe der Rufballinformation, ein Benutzer, insbesondere das medizinische Bedienpersonal, überprüfen, ob der verwendete Patientenrufball für die anstehende Magnetresonanzuntersuchung mit der ausgewählten Magnetresonanzvorrichtung kompatibel ist oder nicht.

In einer vorteilhaften Weiterbildung des erfindungsgemäßen Verfahrens wird vorgeschlagen, dass eine Ausgabeinformation an dem Patientenrufball ausgegeben wird, wobei die Ausgabe der Ausgabeinformation getriggert wird durch eine Aktivierung der Signaleinheit und/oder ein Aussenden eines Registrierungssignals durch die Signaleinheit. Zu einer Ausgabe der Ausgabeinformation weist der Patientenrufball eine Ausgabeeinheit auf. Die Ausgabeeinheit umfasst bevorzugt eine optische Ausgabeeinheit, beispielsweise eine LED-Anzeige. Die Anzeige und/oder Ausgabe der Ausgabeinformation kann beispielsweise durch Änderung einer Farbe der Ausgabeeinheit oder Aufleuchten der Ausgabeeinheit erfolgen. Durch die Ausgabe der Ausgabeinformation direkt an dem Patientenrufball kann der Benutzer, insbesondere ein medizinisches Bedienpersonal, bereits während der Vorbereitung des Patienten für die anstehende Magnetresonanzuntersuchung über die Aktivierung und/oder Registrierung informiert werden. Insbesondere kann das medizinische Bedienpersonal direkt bei dem Einbringen des Patientenrufballs in das Magnetfeld über die Aktivierung und/oder Registrierung informiert werden. Hierdurch kann zudem ein Arbeitsablauf verbessert werden, da das medizinische Bedienpersonal beispielweise direkt über eine fehlgeschlagenen Aktivierung und/oder Registrierung informiert wird und nicht erst an innerhalb des Kontrollraums an der Kommunikationseinheit der Magnetresonanzvorrichtung.

In einer vorteilhaften Weiterbildung des erfindungsgemäßen Verfahrens wird vorgeschlagen, dass die Aktivierung der Signaleinheit ein Aussenden eines Bestätigungssignals durch eine Kommunikationseinheit der Magnetresonanzvorrichtung umfasst, wobei das Bestätigungssignal von der Signaleinheit erfasst wird und an dem Patientenrufball ausgegeben wird. Vorzugsweise erfolgt ein Erfassen des Bestätigungssignals mittels der Signaleinheit, die hierzu eine Empfangseinheit aufweisen kann. Das Aussenden des Bestätigungssignal erfolgt bevorzugt durch die Kommunikationseinheit, die innerhalb des Kontrollraums angeordnet ist und dem medizinischen Bedienpersonal für eine Kommunikation mit dem Patienten zur Verfügung steht. Die Ausgabe des empfangenen Bestätigungssignals erfolgt bevorzugt mittels der Ausgabeeinheit, insbesondere der optischen Ausgabeeinheit, des Patientenrufballs. Die Anzeige und/oder Ausgabe des Bestätigungssignals kann beispielsweise durch Änderung einer Farbe der Ausgabeeinheit oder Aufleuchten der Ausgabeeinheit erfolgen. Das Bestätigungssignal informiert bevorzugt den Benutzer, insbesondere das medizinische Bedienpersonal, über eine erfolgreiche Aktivierung und/oder Registrierung des Patientenrufballs. Durch die Ausgabe der Bestätigungsinformation direkt an dem Patientenrufball kann der Benutzer, insbesondere ein medizinisches Bedienpersonal, bereits während der Vorbereitung des Patienten für die anstehende Magnetresonanzuntersuchung über die Aktivierung und/oder Registrierung informiert werden. Insbesondere kann das medizinische Bedienpersonal direkt bei dem Einbringen des Patientenrufballs in das Magnetfeld über die Aktivierung und/oder Registrierung informiert werden. Hierdurch kann zudem ein Arbeitsablauf verbessert werden, da das medizinische Bedienpersonal beispielweise direkt über eine fehlgeschlagenen Aktivierung und/oder Registrierung informiert wird und nicht erst an innerhalb des Kontrollraums an der Kommunikationseinheit der Magnetresonanzvorrichtung.

In einer vorteilhaften Weiterbildung des erfindungsgemäßen Verfahrens wird vorgeschlagen, dass die Signaleinheit mittels der Aktivierungseinheit deaktiviert wird, sobald ein Wert des erfassten Magnetfelds kleiner ist als ein Deaktivierungsgrenzwert. Derart kann ein hoher Sicherheitsstandard für den Patientenrufball bereitgestellt werden, da dieser deaktiviert wird, sobald er aus dem Magnetfeld entfernt wird und somit auch kein Signal mehr aussenden kann. Der Deaktivierungsgrenzwert für eine Deaktivierung der Signaleinheit kann dabei der gleiche Aktivierungsgrenzwert, der für eine Aktivierung der Signaleinheit verwendet wird, sein. Zudem kann der Deaktivierungsgrenzwert für eine Deaktivierung der Signaleinheit auch unterschiedlich sein zu dem Aktivierungsgrenzwert für eine Aktivierung der Signaleinheit.

In einer vorteilhaften Weiterbildung des erfindungsgemäßen Verfahrens wird vorgeschlagen, dass die Aktivierungseinheit derart ausgebildet ist, dass eine Aktivierung der Signaleinheit und/oder eine Deaktivierung der Signaleinheit einen Verzögerungseffekt umfasst. Durch den Verzögerungseffekt kann verhindert werden, dass der Patientenrufball einer Position im Magnetfeld der Magnetresonanzvorrichtung, wobei die Position einem Wert des Magnetfelds entspricht, der im Bereich des Aktivierungsgrenzwertes und/oder des Deaktivierungsgrenzwertes liegt, entspricht, die Signaleinheit bei geringfügen Positionsänderungen des Patientenrufballs jeweils aktiviert oder deaktiviert wird. Dies kann auch zu einer ressourcenschonenden Verwendung des Patientenrufballs beitragen, da insbesondere die Signaleinheit nicht einer ständigen Aktivierung und Deaktivierung unterliegt. Beispielsweise kann einem Aktivierungsvorgang und/oder einem Deaktivierungsvorgang eine zeitliche Verzögerung überlagert sein, so dass die Aktivierung und/oder Deaktivierung der Signaleinheit einige Zeit nach Überschreiten und/oder Unterschreiten des Grenzwerts erfolgt. Die zeitliche Verzögerung kann dabei einige Sekunden umfassen. Zudem kann die Verzögerung auch dadurch bewirkt werden, dass die beiden Grenzwerte unterschiedlich sind. Ist beispielsweise der Aktivierungsgrenzwert größer als der Deaktivierungsgrenzwert, wird eine aktive Signaleinheit nicht sofort deaktiviert, sobald der erfasste Wert des Magnetfelds den Aktivierungsgrenzwerts unterschreitet. Zudem wird eine deaktive Signaleinheit auch nicht sofort aktiviert, sobald der erfasste Wert des Magnetfelds über dem Deaktivierungsgrenzwert liegt.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung mit einen Patientenrufball in einer schematischen Darstellung,
- Fig. 2: eine Schnitt durch den Patientenrufball und
- Fig. 3: ein Ablaufplan eines Verfahrens zu einem Verwenden eines Patientenrufballs für eine Magnetresonanzuntersuchung.

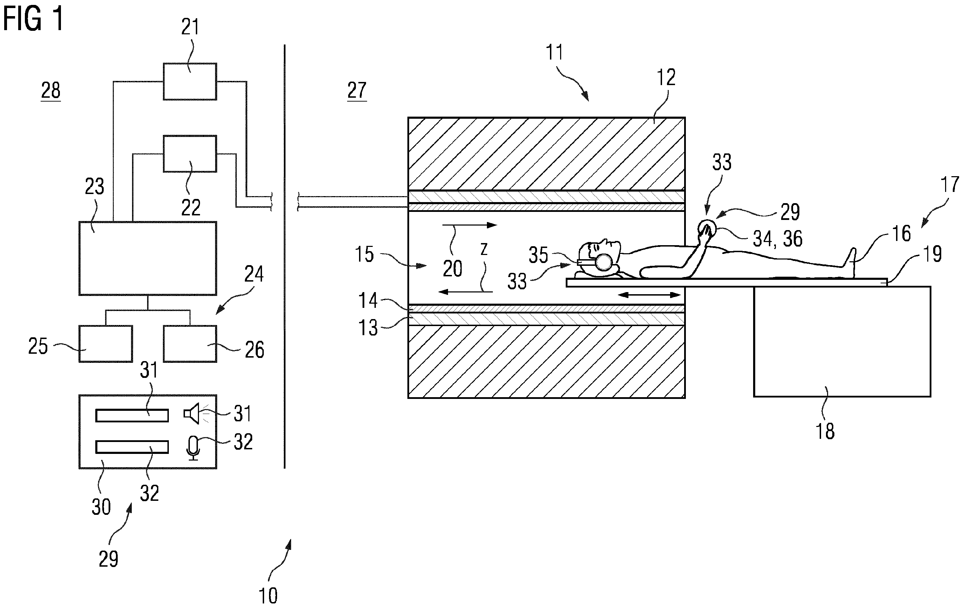

In der Fig. 1 ist eine Magnetresonanzvorrichtung 10 schematisch dargestellt. Die Magnetresonanzvorrichtung 10 umfasst eine als Magneteinheit 11 ausgebildete Scannereinheit mit einem Grundmagneten 12, einer Gradientenspuleneinheit 13 und einer Hochfrequenzantenneneinheit 14. Zudem weist die Magnetresonanzvorrichtung 10 einen Patientenaufnahmebereich 15 auf zu einer Aufnahme eines Patienten 16 für eine Magnetresonanzuntersuchung. Der Patientenaufnahmebereich 15 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 15 jederzeit denkbar.

Für eine Positionierung des Patienten 16, insbesondere eines zu untersuchenden Bereichs des Patienten 16, innerhalb des Patientenaufnahmebereichs 15 weist die Magnetresonanzvorrichtung 10 eine Patientenlagerungsvorrichtung 17 auf. Die Patientenlagerungsvorrichtung 17 weist eine Basiseinheit 18 und einen bezüglich der Basiseinheit 18 bewegbaren Patiententisch 19 auf. Der Patiententisch 19 ist für eine Positionierung des Patienten 16, insbesondere des zu untersuchenden Bereichs des Patienten 16, bewegbar innerhalb des Patientenaufnahmebereichs 15 ausgebildet. Insbesondere ist hierbei der Patiententisch 19 in Richtung einer Längserstreckung des Patientenaufnahmebereichs 15 und/oder in z-Richtung bewegbar gelagert.

Der Grundmagnet 12 der Magneteinheit 11 ist zu einem Erzeugen eines starken und insbesondere konstanten Grundmagnetfelds 20 ausgebildet. Dabei kann der Grundmagnet 12 beispielsweise als supraleitender Grundmagnet 12 oder auch als Dauermagnet ausgebildet sein. Die Gradientenspuleneinheit 13 der Magneteinheit 11 ist zu einer Erzeugung von Magnetfeldgradienten, die für eine Ortskodierung während einer Bildgebung verwendet werden, ausgebildet. Die Gradientenspuleneinheit 13 wird mittels einer Gradientensteuereinheit 21 der Magnetresonanzvorrichtung 10 gesteuert. Die Hochfrequenzantenneneinheit 14 der Magneteinheit 11 ist zu einer Anregung einer Polarisation, die sich in dem von dem Grundmagneten 12 erzeugten Grundmagnetfeld 20 einstellt, ausgebildet. Die Hochfrequenzantenneneinheit 14 wird von einer Hochfrequenzantennensteuereinheit 22 der Magnetresonanzvorrichtung 10 gesteuert und strahlt hochfrequente Magnetresonanzsequenzen in den Patientenaufnahmebereich 15 der Magnetresonanzvorrichtung 10 ein.

Zu einer Steuerung des Grundmagneten 12, der Gradientensteuereinheit 21 und zur Steuerung der Hochfrequenzantennensteuereinheit 22 weist die Magnetresonanzvorrichtung 10 eine Systemsteuereinheit 23 auf. Die Systemsteuereinheit 23 steuert zentral die Magnetresonanzvorrichtung 10, wie beispielsweise das Durchführen einer vorbestimmten bildgebenden Gradientenechosequenz. Zudem umfasst die Systemsteuereinheit 23 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 24, die mit der Systemsteuereinheit 23 verbunden ist. Steuerinformationen wie beispielsweise Bildgebungsparameter, sowie rekonstruierte Magnetresonanzbilder können auf einer Darstellungseinheit 25, beispielsweise auf zumindest einem Monitor, der Benutzerschnittstelle 24 für ein medizinisches Bedienpersonal angezeigt werden. Weiterhin weist die Benutzerschnittstelle 24 eine Eingabeeinheit 26 auf, mittels der Informationen und/oder Parameter während eines Messvorgangs von einem medizinischen Bedienpersonal eingegeben werden können.

Die Scannereinheit 11 der Magnetresonanzvorrichtung 10 ist zusammen mit der Patientenlagerungsvorrichtung 14 innerhalb eines Untersuchungsraums 27 angeordnet. Die Systemsteuereinheit 22 dagegen ist zusammen mit der Benutzerschnittstelle 23 innerhalb eines Kontrollraums 28 angeordnet. Der Kontrollraum 28 ist getrennt von dem Untersuchungsraum 27 ausgebildet. Insbesondere ist der Untersuchungsraum 27 hinsichtlich einer Hochfrequenzstrahlung von dem Kontrollraum 28 abgeschirmt. Während einer Magnetresonanzuntersuchung befindet sich der Patient 13 innerhalb des Untersuchungsraums 27, dagegen befindet sich das medizinische Bedienpersonal innerhalb des Kontrollraums 28.

Für eine Kommunikation zwischen dem Patienten 16 und dem medizinischen Bedienpersonal, beispielsweise ein Arzt, während einer Magnetresonanzuntersuchung weist die Magnetresonanzvorrichtung 10 ein Kommunikationssystem 29 auf. Das Kommunikationssystem 29 umfasst eine erste Kommunikationseinheit 30, die innerhalb des Kontrollraums 28 angeordnet ist und die dem medizinischen Bedienpersonal während der Magnetresonanzuntersuchung für eine Kommunikation mit dem Patienten 16 zur Verfügung steht. Die erste Kommunikationseinheit 30 weist im vorliegenden Ausführungsbeispiel eine Ausgabeeinheit 31 und eine Eingabeeinheit 32 auf. Mittels der Ausgabeeinheit 31 können Informationen vom Patienten 16 an das medizinische Bedienpersonal ausgegeben werden. Die Ausgabeeinheit 31 kann dabei zumindest ein akustisches Ausgabeelement, wie beispielsweise einen Lautsprecher, und/oder zumindest ein visuelles Ausgabeelement, wie beispielsweise ein Display oder ein Monitor, umfassen. Die Eingabeeinheit 32 kann dabei ein Eingabeelement, wie beispielsweise ein Mikrofon und/oder eine Tastatur usw. umfassen.

Das Kommunikationssystem 29 weist zudem eine zweite Kommunikationseinheit 33 auf, die innerhalb des Untersuchungsraums 27 angeordnet ist. Die zweite Kommunikationseinheit 33 steht während einer Magnetresonanzuntersuchung einem Patienten 16 für eine Kommunikation mit dem medizinischen Bedienpersonal zur Verfügung. Bevorzugt umfasst die zweite Kommunikationseinheit 33 eine Eingabeeinheit 34 und eine Ausgabeeinheit 35. Die Ausgabeeinheit 35 umfasst bevorzugt eine akustische Ausgabeeinheit, wie beispielsweise Kopfhörer. Zudem kann die Ausgabeeinheit 35 auch ein visuelle Ausgabeelement, wie beispielsweise ein Display, das innerhalb des Patientenaufnahmebereichs 15 angeordnet ist, umfassen. Die Eingabeeinheit 34 weist ein Eingabeelement, das als Patientenrufball 36 ausgebildet ist, auf.

Die erste Kommunikationseinheit 30 und die zweite Kommunikationseinheit 33 sind zu einem Austausch von Kommunikationssignalen während einer Kommunikation des Patienten 16 mit dem medizinischen Bedienpersonal ausgebildet. Bevorzugt erfolgt die Kommunikation dabei zwischen dem Patientenrufball 36 und der ersten Kommunikationseinheit 30 drahtlos und/oder kabellos. Beispielsweise kann eine kabellose und/oder drahtlose Datenübertragung über eine Funkverbindung, beispielsweise eine Bluetooth-Verbindung, und/oder eine durch Aussenden von Hochfrequenz-Signalen und/oder durch Aussenden von Pilotton-Signalen und/oder weiterer, dem Fachmann als sinnvoll erscheinender kabellosen Datenübertragungsmethoden erfolgen. Die erste Kommunikationseinheit 30 weist hierzu eine Empfangseinheit, die zu einem Empfangen von Signalen des Patientenrufballs 36 ausgebildet ist. Zudem kann die erste Kommunikationseinheit 30 auch eine Sendeeinheit umfassen zu einem Aussenden eines Signals an den Patientenrufball 36.

Die dargestellte Magnetresonanzvorrichtung 10 kann selbstverständlich weitere Komponenten umfassen, die Magnetresonanzvorrichtungen 10 gewöhnlich aufweisen. Eine allgemeine Funktionsweise einer Magnetresonanzvorrichtung 10 ist zudem dem Fachmann bekannt, so dass auf eine detaillierte Beschreibung der weiteren Komponenten verzichtet wird.

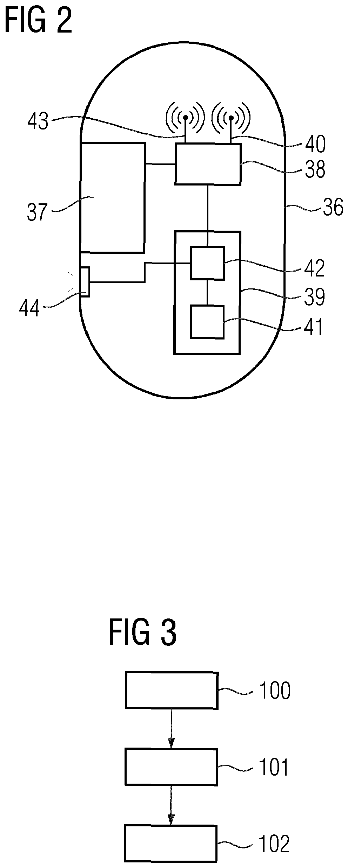

In Fig. 2 ist der Patientenrufball 36 in einer Schnittdarstellung näher dargestellt. Der Patientenrufball 36 weist eine Form und eine Größe auf, dass er von einer Hand des Patienten 16 gehalten und bedient und/oder betätigt werden kann. Bevorzugt ist mit einer Hand des Patienten 16 ein Umgreifen des Patientenrufballs 36, insbesondere ein Umgreifen von mindestens 50% einer Oberfläche des Patientenrufballs 36 und bevorzugt von mindestens 70% einer Oberfläche des Patientenrufballs 36, möglich. Der Patientenrufball 36 umfasst einen Betätigungsbereich 37, eine Signaleinheit 38 und eine Magnetfeldsensoreinheit 39. Der Betätigungsbereich 37 ist zu einem Betätigen, insbesondere zu einem Drücken, mittels einer Hand des Patienten 16 ausgebildet. Der Betätigungsbereich 37 kann dabei eine Taste und/oder einen Drückbereich umfassen.

Die Signaleinheit 38 ist zu einem Aussenden von Signalen ausgebildet. Insbesondere ist die Signaleinheit 38 zu einem Aussenden eines Kommunikationssignals bei einem Drücken des Betätigungsbereichs 37, insbesondere der Taste und/oder des Drückbereichs, ausgebildet. Dabei ist die Signaleinheit 38 zu einem kabellosen und/oder drahtlosen Aussenden von Signalen ausgebildet und weist hierzu eine Sendeeinheit 40 und/oder eine Sendeantenne auf.

Die Magnetfeldsensoreinheit 39 weist einen Magnetfeldsensor 41 und eine Aktivierungseinheit 42 auf. Der Magnetfeldsensor 41 ist zu einem Erfassen eines Werts eines Magnetfelds an dem Ort und/oder an der Position, an der Patientenrufball 36 sich befindet, ausgebildet. Der Magnetfeldsensor 41 kann hierbei eine Hall-Sensor zur Erfassung eines Magnetfelds umfassen. Dabei kann der Hall-Sensor auch einen 3D-Hall-Sensor umfassen. Die Aktivierungseinheit 42 ist bei einem Einbringen des Patientenrufballs 36 in ein Magnetfeld einer Magnetresonanzvorrichtung 10 zu einer Aktivierung der Signaleinheit 38 ausgebildet, wobei eine Aktivierung erst erfolgt, wenn ein von dem Magnetfeldsensor 41 erfasster Wert des Magnetfelds größer ist als ein Aktivierungsgrenzwert.

Des Weiteren ist die Aktivierungseinheit 42 bei einem Entfernen des Patientenrufballs 36 aus einem Magnetfeld der Magnetresonanzvorrichtung 10 zu einer Deaktivierung des Patientenrufballs 36 ausgebildet, wobei eine Deaktivierung erst erfolgt, wenn ein von dem Magnetfeldsensor 41 erfasster Wert des Magnetfelds kleiner ist als ein Deaktivierungsgrenzwert. Der Deaktivierungsgrenzwert für die Deaktivierung der Signaleinheit 38 kann dabei einen gleichen Wert umfassen wie ein Wert des Aktivierungsgrenzwert wie für die Aktivierung der Signaleinheit 38. Zudem können der Deaktivierungsgrenzwert und der Aktivierungsgrenzwert auch unterschiedlich einen gleichen Wert umfassen. Eine Aktivierung der Signaleinheit 38 durch die Aktivierungseinheit 42 kann dabei auch ein Aussenden eines Registrierungssignals durch die Signaleinheit 38 an die erste Kommunikationseinheit 30 umfassen. Zudem kann eine Deaktivierung der Signaleinheit 38 durch die Aktivierungseinheit 42 dabei auch ein Aussenden eines Abmeldesignals durch die Signaleinheit 38 an die erste Kommunikationseinheit 30 umfassen.

Die Aktivierungseinheit 42 weist zu einer Überwachung des Magnetfelds und zur Steuerung der Signaleinheit 38 eine Steuereinheit und/oder eine Recheneinheit auf. Die Steuereinheit und/oder Recheneinheit kann dabei kann dabei eine entsprechende Schaltung und/oder auch ein entsprechendes Rechenmodul aufweisen, die und/oder das zu einer Aktivierung und/oder Deaktivierung der Signaleinheit 38 ausgebildet ist. Bevorzugt erfolgt die Aktivierung und Deaktivierung der Signaleinheit 38 automatisch von der Aktivierungseinheit.

Die Signaleinheit 38 umfasst im vorliegendem Ausführungsbeispiel auch eine Empfangseinheit 43 und/oder eine Empfangsantenne, die zu einem Empfangen eines Bestätigungssignals, das von der ersten Kommunikationseinheit 30 ausgesendet wird, ausgebildet ist.

Der Patientenrufball 36 weist im vorliegendem Ausführungsbeispiel zudem eine Ausgabeeinheit 44 auf. Die Ausgabeeinheit 44 umfasst eine optische Ausgabeeinheit zur optischen und/oder visuellen Ausgabe einer Registrierungsinformation. Die optische Ausgabeeinheit umfasst im vorliegenden Ausführungsbeispiel eine LED-Leuchte, die durch Aufleuchten und/oder durch eine Farbänderung einer Lichtanzeige die Registrierungsinformation, insbesondere eine erfolgreiche Registrierung und/oder Aktivierung, des Patientenrufballs 36 dem medizinischen Bedienpersonal und/oder dem Patienten 16 anzeigt. In einer alternativen Ausgestaltung der Ausgabeeinheit 44 kann diese auch eine akustische Ausgabeeinheit und/oder eine haptische Ausgabeeinheit umfassen. In einer alternativen Ausgestaltung des Patientenrufballs 36 kann dieser auch keine Ausgabeeinheit 40 umfassen.

Der dargestellte Patientenrufball 36 kann selbstverständlich weitere Komponenten umfassen, die Patientenrufbälle 36 gewöhnlich aufweisen. Eine allgemeine Funktionsweise eines Patientenrufballs 36 ist zudem dem Fachmann bekannt, so dass auf eine detaillierte Beschreibung der weiteren Komponenten verzichtet wird.

In Fig. 3 ist ein Verfahren zu einem Verwenden eines Patientenrufballs 36 für eine Magnetresonanzuntersuchung schematisch dargestellt. Hierbei wird zunächst in einem ersten Verfahrensschritt 100 ein Wert des Magnetfelds der Magnetresonanzvorrichtung 10 am Patientenrufball 36 mittels des Magnetfeldsensors 41 des Patientenrufballs 36 erfasst und an die Aktivierungseinheit 42 weitergeleitet. Mittels des Magnetfeldsensors 41 wird permanent ein Wert des Magnetfelds am Ort des Patientenrufballs 36 erfasst und an die Aktivierungseinheit 42 weitergeleitet.

In einem zweiten Verfahrensschritt 101 erfolgt ein Aktivieren der Signaleinheit 38 des Patientenrufballs 36 mittels der Aktivierungseinheit 42, sobald der Wert des erfassten Magnetfelds einen Aktivierungsgrenzwert überschreitet. Hierbei werden von der Aktivierungseinheit 42 die erfassten Werte des Magnetfelds permanent mit dem Aktivierungsgrenzwert verglichen. Der Aktivierungsgrenzwert kann hierbei derart ausgebildet sein, dass erst bei einem Einfahren des Patiententischs 19 zusammen mit dem Patientenrufball 36 in den Patientenaufnahmebereich 15 der erfasste Wert des Magnetfelds den Aktivierungsgrenzwert überschreitet, so dass außerhalb des Patientenaufnahmebereichs 15 oder in größerer Entfernung zu der Scannereinheit, beispielsweise ein Abstand zur Scannereinheit von mehr als 2 m, keine Aktivierung der Signaleinheit 38 erfolgen kann.

Die Aktivierung der Signaleinheit 38 durch die Aktivierungseinheit 42 umfasst in diesem zweiten Verfahrensschritt 101 zudem ein Aussenden eines Registrierungssignals durch die Signaleinheit 38. Hierbei wird, sobald der erfasste Wert des Magnetfelds über den Aktivierungsgrenzwert liegt, ein Registrierungssignal von der Signaleinheit 38 ausgesendet. Eine Steuerung des Generierens und/oder Aussendens des Registrierungssignals erfolgt hierbei mittels der Aktivierungseinheit 42. Das Registrierungssignal wird von der ersten Kommunikationseinheit 30 erfasst und somit der ersten Kommunikationseinheit 30 signalisiert, dass der Patientenrufball 36 sich nun in einem aktiven Betriebsmodus befindet.

In einer alternativen Ausgestaltung des Patientenrufballs 36 und/oder des Verfahrens zu einem Verwenden des Patientenrufballs 36 für eine Magnetresonanzuntersuchung kann es auch vorgesehen sein, dass kein Registrierungssignal von der Signaleinheit 38 an die erste Kommunikationseinheit 30 gesendet wird.

Zusätzlich zu dem Registrierungssignal wird im vorliegenden Ausführungsbeispiel eine Rufballinformation an die erste Kommunikationseinheit 30 der Magnetresonanzvorrichtung 10 übermittelt. Die Rufballinformation wird hierbei ebenfalls von der Signaleinheit 38 an die erste Kommunikationseinheit 30 gesendet. Die Rufballinformation umfasst bevorzugt eine Typennummer und/oder Seriennummer des Patientenrufballs 36. Zudem kann die Rufballinformation weitere Informationen bezüglich des Patientenrufballs 36 umfassen. Anhand der Rufballinformation kann die erste Kommunikationseinheit 30 überprüfen, ob der verwendete Patientenrufball 36 mit der Magnetresonanzvorrichtung 10 kompatibel ist oder für eine Verwendung mit der Magnetresonanzvorrichtung 10 zugelassen ist.

In einer alternativen Ausgestaltung des Patientenrufballs 36 und/oder des Verfahrens zu einem Verwenden des Patientenrufballs 36 für eine Magnetresonanzuntersuchung kann es auch vorgesehen sein, dass keine Rufballinformation von der Signaleinheit 38 an die erste Kommunikationseinheit 30 gesendet wird.

Die Aktivierung des Patientenrufballs 36, insbesondere der zweite Verfahrensschritt 101, umfasst zudem eine Ausgabe einer Registrierungsinformation und/oder der Rufballinformation an der ersten Kommunikationseinheit 30, insbesondere mittels der Ausgabeeinheit 31 der ersten Kommunikationseinheit 30. Die Registrierungsinformation umfasst bevorzugt eine Ausgabe, die eine erfolgreiche Registrierung und/oder Aktivierung des Patientenrufballs 36 an der ersten Kommunikationseinheit 30 für das medizinische Bedienpersonal signalisiert. Beispielsweise kann ein Symbol eines Patientenrufballs auf einem Display und/oder Monitor der ersten Kommunikationseinheit 30 eingeblendet werden. Zudem kann ein bereits angezeigtes Symbol eines Patientenrufballs auf einem Display und/oder Monitor der ersten Kommunikationseinheit 30 in einer anderen Farbe dargestellt und/oder angezeigt werden.

Die Aktivierung des Patientenrufballs 36, insbesondere der zweite Verfahrensschritt 101, umfasst im vorliegenden Ausführungsbeispiel zudem ein Aussenden eines Bestätigungssignals durch die erste Kommunikationseinheit 30 an den Patientenrufball 36. Das Bestätigungssignal wird von der Signaleinheit 38, insbesondere der Empfangseinheit 43 der Signaleinheit 38, des Patientenrufballs 36 erfasst. Dieses Bestätigungssignal signalisiert der Aktivierungseinheit 42, dass eine Aktivierung und/oder Registrierung des Patientenrufballs 36 an der ersten Kommunikationseinheit 30 erfolgt ist.

In einer alternativen Ausgestaltung des Patientenrufballs 36 und/oder des Verfahrens zu einem Verwenden des Patientenrufballs 36 für eine Magnetresonanzuntersuchung kann es auch vorgesehen sein, dass keine Bestätigungssignal von der ersten Kommunikationseinheit 30 gesendet wird.

Zudem umfasst die Aktivierung des Patientenrufballs 36, insbesondere der zweite Verfahrensschritt 101, im vorliegenden Ausführungsbeispiel, dass eine Ausgabeinformation an dem Patientenrufball 36, insbesondere an der Ausgabeeinheit 44 des Patientenrufballs 36, ausgegeben wird. Die Ausgabeinformation informiert dabei einen Benutzer, insbesondere das medizinische Bedienpersonal und/oder den Patienten, über eine erfolgreiche Aktivierung und/oder Registrierung des Patientenrufballs 36 an der ersten Kommunikationseinheit 30. Die Ausgabe der Ausgabeinformation wird dabei getriggert durch die Aktivierung der Signaleinheit 38 oder durch ein Aussenden eines Registrierungssignals durch die Signaleinheit 36 oder durch ein Empfangen des Bestätigungssignals.

In einem weiteren, dritten Verfahrensschritt 102 ist die Aktivierungseinheit 42 dazu ausgebildet, die Signaleinheit 38 zu deaktivieren, sobald ein Wert des erfassten Magnetfelds am Ort des Patientenrufballs 36 kleiner ist als ein Deaktivierungsgrenzwert. Dies ist insbesondere der Fall, wenn die Magnetresonanzuntersuchung beendet ist und der Patient 16 zusammen mit dem Patientenrufball 36 den Patientenaufnahmebereich 15 der Scannereinheit verlässt. In diesem Verfahrensschritt 102 kann die Signaleinheit 38 auch zu einem Aussenden eines Deaktivierungssignals an die erste Kommunikationseinheit 30 ausgebildet sein. Eine Deaktivierung der Signaleinheit 38 kann dabei auch an der Ausgabeeinheit 31 der ersten Kommunikationseinheit 30 angezeigt werden. Beispielsweise kann ein bereits angezeigtes Symbol eine Patientenrufballs auf einem Display und/oder Monitor ausgeblendet werden. Zudem kann ein bereits angezeigtes Symbol eines Patientenrufballs auf einem Display und/oder Monitor der ersten Kommunikationseinheit 30 in einer anderen Farbe dargestellt und/oder angezeigt werden.

In einer alternativen Ausgestaltung des Patientenrufballs 36 und/oder des Verfahrens zu einem Verwenden des Patientenrufballs 36 für eine Magnetresonanzuntersuchung kann es auch vorgesehen sein, dass kein Deaktivierungssignal von der Signaleinheit 38 ausgesendet wird.

Im vorliegenden Ausführungsbeispiel ist es zudem vorgesehen, dass eine Aktivierung und/oder eine Deaktivierung der Signaleinheit 38 durch die Aktivierungseinheit 42 einen Verzögerungseffekt umfasst. Beispielsweise kann dem Aktivierungsvorgang und/oder dem Deaktivierungsvorgang eine zeitliche Verzögerung überlagert sein, so dass die Aktivierung und/oder Deaktivierung der Signaleinheit 38 einige Zeit nach Überschreiten des Aktivierungsgrenzwerts und/oder Unterschreiten des Deaktivierungsgrenzwerts erfolgt. Die zeitliche Verzögerung kann dabei einige Sekunden umfassen. Zudem kann die Verzögerung auch dadurch bewirkt werden, dass die beiden Grenzwerte unterschiedlich sind. Ist beispielsweise der Aktivierungsgrenzwert größer als der Deaktivierungsgrenzwert, wird eine aktive Signaleinheit 38 nicht sofort deaktiviert, sobald der erfasste Wert des Magnetfelds den Aktivierungsgrenzwerts unterschreitet. Zudem wird eine deaktive Signaleinheit 38 auch nicht sofort aktiviert, sobald der erfasste Wert des Magnetfelds über dem Deaktivierungsgrenzwert liegt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Patientenrufball, der zu einer Kommunikation eines Patienten während einer medizinischen Magnetresonanzuntersuchung mit einem medizinischen Bedienpersonal ausgebildet ist, umfassend einen Betätigungsbereich, der zu einem Betätigen, insbesondere zu einem Drücken, durch einen Patienten ausgebildet ist, und eine Signaleinheit, die dazu ausgebildet ist, bei einem Drücken des Betätigungsbereichs ein Kommunikationssignal auszusenden,
**gekennzeichnet durch** eine Magnetfeldsensoreinheit mit einem Magnetfeldsensor und einer Aktivierungseinheit, wobei die Aktivierungseinheit zu einer Aktivierung der Signaleinheit ausgebildet ist, sobald ein von dem Magnetfeldsensor erfasster Wert eines Magnetfelds größer ist als ein Aktivierungsgrenzwert.

2. Patientenrufball nach Anspruch 1,
**dadurch gekennzeichnet, dass** die Signaleinheit zu einem Aussenden zumindest eines Registrierungssignals ausgebildet ist.

3. Patientenrufball nach einem der vorhergehenden Ansprüche,
**gekennzeichnet durch** eine Ausgabeeinheit, die zu einer Ausgabe einer Registrierungsinformation ausgebildet ist.

4. Patientenrufball nach einem der vorhergehenden Ansprüche,
**dadurch gekennzeichnet, dass** die Signaleinheit eine Empfangseinheit zu einem Empfangen eines Bestätigungssignals aufweist.

5. Patientenrufball nach einem der vorhergehenden Ansprüche,
**dadurch gekennzeichnet, dass** die Aktivierungseinheit zu einer Deaktivierung der Signaleinheit ausgebildet ist, sobald ein von dem Magnetfeldsensor erfasster Wert eines Magnetfeld kleiner ist als ein Deaktivierungsgrenzwert.

6. Magnetresonanzvorrichtung mit einer ersten Kommunikationseinheit, die innerhalb eines Kontrollraums angeordnet ist, und einer zweiten Kommunikationseinheit, die innerhalb eines Untersuchungsraums angeordnet ist, wobei die zweite Kommunikationseinheit einen Patientenrufball nach einem der vorhergehenden Ansprüche aufweist und wobei die erste Kommunikationseinheit und der Patientenrufball zu einem Austausch von Kommunikationssignalen ausgebildet sind.

7. Magnetresonanzvorrichtung nach Anspruch 6,
**dadurch gekennzeichnet, dass** die erste Kommunikationseinheit eine Empfangseinheit, die zu einem Empfangen von Signalen des Patientenrufballs ausgebildet ist, und eine Sendeeinheit, die zu einem Senden von Signalen an den Patientenrufball ausgebildet ist, umfasst.

8. Verfahren zu einem Verwenden eines Patientenrufballs für eine Magnetresonanzuntersuchung mit einem Patientenrufball nach einem der Ansprüche 1 bis 5 und einer Magnetresonanzvorrichtung nach einem der Ansprüche 6 bis 7, wobei das Verfahren die folgenden Verfahrensschritte umfasst:
- Erfassen eines Werts des Magnetfelds am Patientenrufball mittels eines Magnetfeldsensors des Patientenrufballs und
- Aktivieren einer Signaleinheit des Patientenrufballs mittels einer Aktivierungseinheit, sobald der erfasste Wert des Magnetfelds einen Aktivierungsgrenzwert überschreitet.

9. Verfahren nach Anspruch 8,
**dadurch gekennzeichnet, dass** die Aktivierung der Signaleinheit ein Aussenden eines Registrierungssignals durch die Signaleinheit umfasst, wobei das Registrierungssignal von einer Kommunikationseinheit der Magnetresonanzvorrichtung erfasst wird.

10. Verfahren nach einem der Ansprüche 8 oder 9,
**dadurch gekennzeichnet, dass** zusätzlich zu dem Registrierungssignal eine Rufballinformation an die Kommunikationseinheit der Magnetresonanzvorrichtung übermittelt wird.

11. Verfahren nach einem der Ansprüche 9 und/oder 10,
**dadurch gekennzeichnet, dass** eine Registrierungsinformation und/oder die Rufballinformation an der Kommunikationseinheit der Magnetresonanzvorrichtung ausgegeben wird.

12. Verfahren nach einem der Ansprüche 8 bis 11,
**dadurch gekennzeichnet, dass** eine Ausgabeinformation an dem Patientenrufball ausgegeben wird, wobei die Ausgabe der Ausgabeinformation getriggert wird durch eine Aktivierung der Signaleinheit und/oder ein Aussenden eines Registrierungssignals durch die Signaleinheit.

13. Verfahren nach einem der Ansprüche 8 bis 12,
**dadurch gekennzeichnet, dass** die Aktivierung der Signaleinheit ein Aussenden eines Bestätigungssignals durch eine Kommunikationseinheit der Magnetresonanzvorrichtung umfasst, wobei das Bestätigungssignal von der Signaleinheit erfasst wird und an dem Patientenrufball ausgegeben wird.

14. Verfahren nach einem der Ansprüche 8 bis 13,
**dadurch gekennzeichnet, dass** von der Aktivierungseinheit die Signaleinheit deaktiviert wird, sobald ein Wert des erfassten Magnetfelds kleiner ist als ein Deaktivierungsgrenzwert.

15. Verfahren nach einem der Ansprüche 8 bis 14,
**dadurch gekennzeichnet, dass** die Aktivierungseinheit derart ausgebildet ist, dass die Aktivierung der Signaleinheit und/oder die Deaktivierung der Signaleinheit einen Verzögerungseffekt umfasst.
